(19) Europäisches Patentamt
European Patent Office
Office européen des brevets

(11) **EP 3 669 408 B1**

(12) **EUROPEAN PATENT SPECIFICATION**

(45) Date of publication and mention of the grant of the patent:
**12.05.2021 Bulletin 2021/19**

(21) Application number: **18782295.2**

(22) Date of filing: **15.08.2018**

(51) Int Cl.:
*H01M 50/531* (2021.01)     *G01N 27/403* (2006.01)
*H01M 8/16* (2006.01)     *H01M 8/20* (2006.01)
*H01M 6/22* (2006.01)     *H01M 6/34* (2006.01)
*H01M 6/40* (2006.01)     *A61B 5/145* (2006.01)
*A61B 5/1486* (2006.01)

(86) International application number:
**PCT/CZ2018/050044**

(87) International publication number:
**WO 2019/034189 (21.02.2019 Gazette 2019/08)**

(54) **GALVANIC SYSTEM WITH INCREASED OUTPUT VOLTAGE AND WAY OF INCREASING THE OUTPUT VOLTAGE OF THE GALVANIC SYSTEM**

GALVANISCHES SYSTEM MIT ERHÖHTER AUSGANGSSPANNUNG UND WEGE ZUR ERHÖHUNG DER AUSGANGSSPANNUNG DES GALVANISCHEN SYSTEMS

SYSTÈME GALVANIQUE AVEC TENSION DE SORTIE ACCRUE ET MANIÈRE D'AUGMENTER LA TENSION DE SORTIE DU SYSTÈME GALVANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2017 CZ 20170472**

(43) Date of publication of application:
**24.06.2020 Bulletin 2020/26**

(73) Proprietor: **Masarykova Univerzita
602 00 BRNO 2 (CZ)**

(72) Inventors:
• **LACINA, Karel
621 00 Brno (CZ)**
• **SOPOUSEK, Jakub
628 00 Brno (CZ)**
• **KONHEFR, Martin
377 01 Jindrichuv Hradec (CZ)**

(74) Representative: **Hartvichova, Katerina
HARBER IP s.r.o.
Dukelskych hrdinu 567/52
170 00 Praha 7 (CZ)**

(56) References cited:
**US-A- 3 752 704     US-A1- 2010 099 010**

• BIRTE JACHE ET AL: "Copper sulfides for rechargeable lithium batteries: Linking cycling stability to electrolyte composition", JOURNAL OF POWER SOURCES, vol. 247, 12 September 2013 (2013-09-12), pages 703-711, XP055536677, CH ISSN: 0378-7753, DOI: 10.1016/j.jpowsour.2013.08.136
• ALON SZCZUPAK ET AL: "Living battery - biofuel cells operating in vivo in clams", ENERGY & ENVIRONMENTAL SCIENCE, vol. 5, no. 10, 12 April 2012 (2012-04-12), page 8891, XP055536608, Cambridge ISSN: 1754-5692, DOI: 10.1039/c2ee21626d

EP 3 669 408 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The invention relates to a galvanic system with increased output voltage and to the connection of several galvanic cells in one volume of the electrolyte respectively, and to the method of increasing the output voltage of the galvanic system by connecting several galvanic cells in one volume of the electrolyte.

Background Art

Nowadays, a variety of galvanic cells, primary or secondary (accumulators), and batteries can be used as power sources. All such sources of energy work on the principle of two complementary electrochemical reactions (oxidation and reduction) that run spontaneously to provide energy tahat can further be used. The voltage of a galvanic cell is given by the difference of the potentials of the respective half-cell redox reactions. The half-cells constitute of the interface between the respective electrolyte and the electrode - the anolyte / anode and the catholyte / cathode. The well known type of galvanic cell is the Daniell cell, which is composed of a copper cathode immersed in a $CuSO_4$ solution and a zinc anode immersed in a $ZnSO_4$ solution. In this type of cell, the following electrode reactions occur: reduction of $Cu^{2+}$ ions on the cathode and oxidation of the metal anode Zn to $Zn^{2+}$ ions.

In all types of galvanic cells, the maximum possible voltage is determined by the thermodynamic voltage of the half-cell reactions, i.e., for the Daniell cell, the maximum voltage is given by the reactions:

$$Zn(s) \rightarrow Zn^{2+}(aq) + 2e^-, \qquad E = -0.7618 \text{ V}$$

$$Cu^{2+}(aq) + 2e^- \rightarrow Cu(s), \qquad E = +0.340 \text{ V}$$

The full reaction is:

$$Zn(s) + Cu^{2+}(aq) \rightarrow Zn^{2+}(aq) + Cu(s) \qquad E_{cell} = 1.1018 \text{ V}$$

Similar sources of energy also include fuel or biofuel cells, where the oxidized or reduced material is a particular fuel - e.g. oxidation of hydrogen on the anode and reduction of oxygen on the cathode. In the case of biofuel cells, the "fuel" is catalytically treated by biological molecules (e.g., enzymes) present in the electrolyte (in the vessel) of the respective electrode or more frequently immobilized on the surface of one of the electrodes. This category also includes a source of energy based on the generation of energy through whole organisms, cell cultures. These are the so-called microbial fuel cells.

Applications which require voltage that is higher than the thermodynamic voltage $E_{cell}$ require multiple cells to be connected at the same time. Batteries are the most commonly used for this purpose. The battery is the set of series-connected galvanic cells with separate electrolytes. A similar approach is also used for (bio)fuel or microbial cells.

However, there are also applications, where multiple galvanic cells have to be connected into one system in series to achieve higher output voltage, but the connection with separate electrolytes is not possible since only one volume of electrolyte is available. However, in such an arrangement, the general effect of an electrolytic short-circuit applies, i.e. voltage is reduced to the initial thermodynamic voltage given by the connected half-cell reactions. Thus, the electrolyte, which is common to all the inserted cells, short-circuits the inserted cells. Only the electrode reactions (voltage) of the "outer" (main) electrodes, which provide the output voltage, occur.

The construction of (bio)fuel cells would generally be much easier when connecting all cells in a common volume of electrolyte. There would be no need to address the technically and spatially problematic electrolytic interconnection of these cells, i.e., conductive electrolyte connection. Possible applications include, for example, also various technologies of implantable biofuel cells using "fuel" within a given organism, working in a common volume of electrolyte. Here, it is not possible to separate individual galvanic cells from each other since the body itself represents one volume of electrolyte.

Disclosure of the invention

The above-mentioned technical problem is solved by the galvanic system according to claim 1.

The object of the invention is to connect one or more galvanic short-circuits between an anode and a cathode (main pair), wherein the whole system is embedded in one volume of the electrolyte. The term "galvanic short-circuit connection" is to be understood as the insertion of a second (or further) pair of anode-cathode (inner electrodes) between the main pair of electrodes such that an inner cathode is located next to the main anode, and wherein the inner electrodes are located at a certain distance for minimizing electrolytic short-circuit. Furthermore, the inner electrodes are connected by a conductor of higher conductivity than the electrolyte used, which may or may not be isolated from the electrolyte. In this way, it is possible to increase the output voltage of the system by combining several galvanic cells even in one volume of the electrolyte, without significantly inducing the electrolytic short-circuit which reduces the resulting output voltage.

If the electrodes are sufficiently distant from each other, the electrolytic short-circuit effect does not apply and both cells, or all connected cells, respectively, work separately so that a corresponding multiple of the thermodynamic voltage of the galvanic cell is delivered. The multiple of the thermodynamic voltage is given by the number of connected cells and their relative distance, wherein the maximum possible multiple of the thermodynamic voltage occurs at the so-called saturation distance.

For one short-circuit, the saturation distance is defined as a short-circuit length, the further increase of which will no longer lead to a significant increase in the output voltage, or, where the output voltage reaches 95% of the voltage that would be achieved if the length of the short-circuit (distance between the inner anode and the inner cathode) was infinitely large. Further extension of the distance between the electrodes of the short-circuit does not affect the output voltage of the cell, i. e., in an ideal case, for two cells, i. e. one cell with one galvanic short-circuit, the output voltage is twice the initial thermodynamic voltage of the basic cell ($2^*E_{cell}$). Under optimal conditions, it is 95% of twice the thermodynamic voltage of a single galvanic cell, i. e. for one short-circuit, 95% of three times the thermodynamic voltage for two short-circuits, etc. When connecting multiple cells, the distance of each galvanic cell from the next closest galvanic cell is evaluated.

The effect of the electrolytic short-circuit is, in addition to the distance of the inner electrodes, influenced by other factors, such as system properties. This includes mainly the electrolyte conductivity (electrolyte resistance, $R_{sol}$) and the concentration of substances used in the half-cell reactions (fuel in fuel cells). For example, the saturation distance increases with the increasing concentration of electrolyte ions, or, while maintaining the length of the short-circuit, the increasing concentration of ions in the electrolyte decreases the output voltage. The output voltage also decreases with decreasing concentrations of substances applied in the half-cell reactions (fuel in the fuel cells).

These effects can be used to increase the output voltage. With the constant distance of the inner cells, i.e. the constant length of the galvanic short-circuit, the output voltage of the multiple cell (cell with one or more galvanic short-circuits) increases with the decreasing conductivity of the electrolyte and the increasing concentration of the substances applied in the half-cell reactions (fuel in the fuel cells). Since the decrease of the electrolyte conductivity is undesirable due to the subsequent reduction of the current densities, the higher voltage is preferably provided by increasing the concentration of the substances applied in the half-cell reactions, or of the fuel in fuel cells, respectively, while the distance of the inner cells remains constant, i. e., the length of the galvanic short-circuit remains constant, wherein it is understood that increasing concentration means using any higher concentration while maintaining the other parameters. There is no particular reference value of the increase of the concentration, because the concentration of substances can be changed and adjusted and no reference concentration values are given.

The galvanic system of multiple cells of the present invention comprises at least two galvanic cells, each of which comprises a pair of anode-cathode electrodes. The number of cells that can in this way be connected into a common volume of electrolyte is limited only by a spatial size of the system.

An aqueous solution, a gel, an ionic liquid or other suitable medium may be used as an electrolyte. In a preferred embodiment, the electrolyte may be, for example, a filter paper that has been pre-impregnated with a suitable electrolyte solution and subsequently dried so that the electrolyte is in solid form without a solvent. In this case, the system is "activated" only when the filter paper is impregnated with water or other liquid medium, in which the electrolyte (e.g., the crystalline substance) dissolves. The advantage of such a system is that as long as the contact with moisture (steams of other liquid medium) is avoided, the system can be retained as a source of energy, which, in principle, remains charged for unlimited period of time, becasue ther is no spontaneous discharge. In addition to the filter paper; the electrolyte carrier may comprise any material which allows the above steps of electrolyte-impregnation and drying, e.g., a fabric or a polymeric porous matrix.

In another aspect of the present invention, a method of increasing the output voltage of a galvanic system by combining several cells in a common volume of electrolyte is provided according to claim 5.

In a preferred embodiment with several galvanic short-circuits, the connection of short-circuits is as follows: an inner cathode of the first galvanic short-circuit (the first inner cathode) is placed between the main anode and the main cathode close to the main anode. Further, the first inner anode is placed between the first inner cathode and the main cathode at a distance from the first inner cathode that is greater than or equal to the saturation distance. The first inner cathode and the first inner anode are connected with a conductor of higher conductivity than the conductivity of the electrolyte. The second galvanic short-circuit is connected such that an inner cathode of the second galvanic short-circuit (second inner cathode) is placed between the first inner anode and the main cathode close to the first inner anode and a second inner anode is placed between the second inner cathode and the main cathode. The second inner cathode and the second inner anode are connected with a conductor of higher conductivity than the conductivity of the electrolyte. Any further galvanic short-circuit is connected in an analogous manner.

The above description of the galvanic system with the connection of several galvanic cells and the method of increasing of the voltage of such a system applies analogously also to the fuel and biofuel cells. The electrical voltage obtained from the thermodynamic voltages of the chemical reactions on the respective electrodes can be increased by connecting one or more pairs of electrodes into a common volume of the electrolyte. In the basic setup, fuel is processed on the anode (e.g. hydrogen or borohydride) and an oxidant (e.g. oxygen or chlorine dioxide) is processed on the cathode.

In the case of biofuel cells, chemical reactions carried out in living organisms or catalyzed by biological systems or by their components are used. This especially includes systems where an enzyme or other biologically active substance catalyzing the conversion of the substrate to a product useable as a fuel in the respective half-cell reaction on the electrode is dissolved in the electrolyte solution. These active substances may also be immobilized on the respective electrodes so that the generated product is directly converted by the respective electrode. This group also includes microbial fuel cells, where the product of biological processes is used as a fuel processed on the respective electrode. In this case, the electrode system is placed into common cell culture volume, analogously to the galvanic cell solution. It is possible to immobilize the cell cultures to the surface of the respective electrode, on the surface of which, the fuel is converted into electrical energy.

In yet another aspect of the present invention, a device for measuring body functions or parameters is provided according to claim 9.

The basis of such a device may, for example, be a biofuel cell, the anode of which may be implemented as a carbon nanotube aggregate on a gold electrode with an immobilized enzyme PQQ-dependent glucose dehydrogenase catalyzing glucose oxidation and the cathode of which may be implemented as a carbon nanotube aggregate on a gold electrode modified by oxygen-reducing laccase enzyme. The so-formed pairs of anode-cathode electrodes of the outer and inner cells may then be integrated as a source of energy for a device - a (bio) sensor for measuring various parameters or body functions (e.g., a glucometer). The device is then implanted into the patient's body. Since the body fluids of the patient provide the electrolyte in this embodiment, the biofuel cell becomes active only after implantation.

Brief description of figures

Fig. 1 - schematic view of a galvanic system based on a Daniell cell; galvanic system with one galvanic short-circuit in one volume of electrolyte

Fig. 2 - schematic view of the length of the galvanic short-circuit, the relation between the length of the galvanic short-circuit and the saturation distance

Fig. 3 - dependency of the change of the output voltage on the length of the galvanic short-circuit, respectively on the distance of the electrodes of the galvanic short-circuit based on a Daniell cell - a combination of copper and zinc electrodes in the environment of 1 mM of $CuSO_4$

Fig. 4 - schematic view of a device for testing of the effect of an electrolytic short-circuit on the length of a galvanic short-circuit

Fig. 5 - dependency of the change of the output voltage on the length of the galvanic short-circuit, respectively on the distance of the electrodes of the galvanic short-circuit based on the Daniellcell - a combination of copper and zinc electrodes in the environment of different concentrations of $CuSO_4$

Fig. 6 - schematic view of a galvanic system with one (a), two (a + b) and three (a + b + c) galvanic short-circuits

Fig. 7 - dependency of the change of the output voltage of the galvanic system on the number of galvanic short-circuits connected in the system

Fig. 8 - the dependency of the increase of the output voltage of the galvanic system conaining the $MnO_2$ cathode and the zinc anode with the connection of the corresponding galvanic short-circuit

Fig. 9 - schematic view of a proposed power supply device according to the present invention utilizing an electrolyte carrier that has been pre-impregnated with a suitable electrolyte solution and subsequently dried so that activation (energy delivery) occurs after the addition of a liquid

Fig. 10 - schematic view of the assembling process of the power supply device of Fig. 9

## Examples

### Example 1

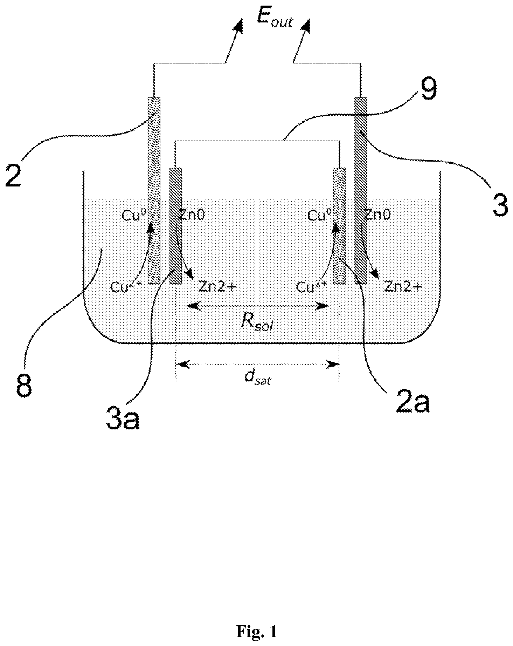

A basic embodiment of the invention is represented by the connection of two galvanic cells in a common volume of electrolyte according to Fig. 1, wherein the connected galvanic cells are based on the basic Daniell cell, i.e. they contain copper cathodes 2, 2a and zinc anodes 3, 3a. The electrolyte 8 in this embodiment is a $CuSO_4$ solution, and it is common to both connected cells, or for all electrodes, respectively. The copper cathode 2 and the zinc anode 3 form the main pair of electrodes, both of which are immersed in the $CuSO_4$ solution. A pair of inner electrodes - a cathode 2a and the anode 3a is immersed between the main electrodes in the same $CuSO_4$ solution so that the inner zinc anode 3a is positioned close to the main copper cathode 2 and an inner copper cathode 2a is positioned close to the main zinc anode 3. The inner electrodes are interconnected by a conductor 9 of higher conductivity then the conductivity of electrolyte 8, e.g., by a metal conductor.

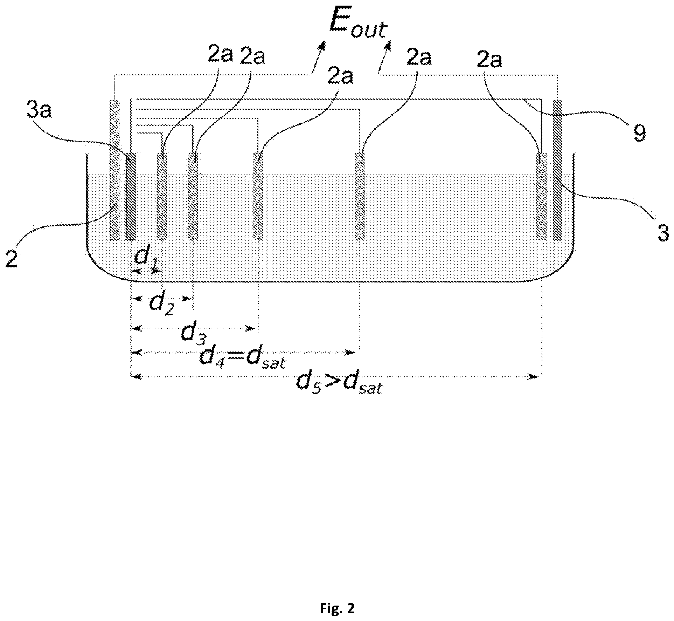

In Fig. 1, the saturation distance is denoted as $d_{sat}$ - distance of the galvanic short-circuit, i.e. the distance of the inner electrodes cathode - 2a, anode - 3a. The arrangement of the main pair electrodes and the arrangement of the inner electrodes of the galvanic short-circuit is further illustrated in Fig. 2. The distance of the inner electrodes $d$ is the factor which, for a given number of connected cells, affects the output voltage $E_{out}$ to the largest extent. In this schematic representation, for a single connected galvanic short-circuit, the distances $d_1$, $d_2$, $d_3$ represent distances, at which the effect of electrolytic short-circuit still significantly applies (e.g. $d_1$ ($E_{out} \approx E_{cell}$), $d_2$ ($E_{out} \approx E_{cell}$)), so that the increase of the voltage does not exceed, for example, 10% of the initial voltage, or at which the output voltage reaches values higher than the initial thermodynamic voltage of the half-cell reactions, but the voltage increase does not reach the maximum yet (e.g. $d_3$ ($E_{out} > Ec_{ell}$)).

The maximal values of the increase of the output voltage are reached at such a length of the galvanic short-circuit, where $d \geq d_{sat}$ (e.g. $d_4 = d_{sat}$ ($E_{out} \to 2*E_{cell}$)). The saturation distance $d_{sat}$ is defined as the length of the short-circuit, the further increase of which does not lead to further significant increase of the output voltage (e.g. $d_5 > d_{sat}$($E_{out} \to 2*E_{cell}$)), where the output voltage reaches the value of 95% of the voltage that would be achieved if the length of the short-circuit was infinitely large. Thus, the distance of the inner electrodes, at which the effect of the electrolytic short-circuit still significantly applies, as well as the saturation distance are experimentally measurable by measuring of the output voltage of the system for different distances of the galvanic short-circuit, wherein the measurement can be done for any type of a galvanic cell. The increase of the output voltage thus occurs at the distance of a galvanic short-circuit, which is greater than the distance of the galvanic short-circuit, at which the electrolytic short-circuit still significantly applies (the output voltage approaches the initial thermodynamic voltage), wherein the maximum increase is achieved at the length of the galvanic short-circuit that is greater than or equal to the saturation distance.

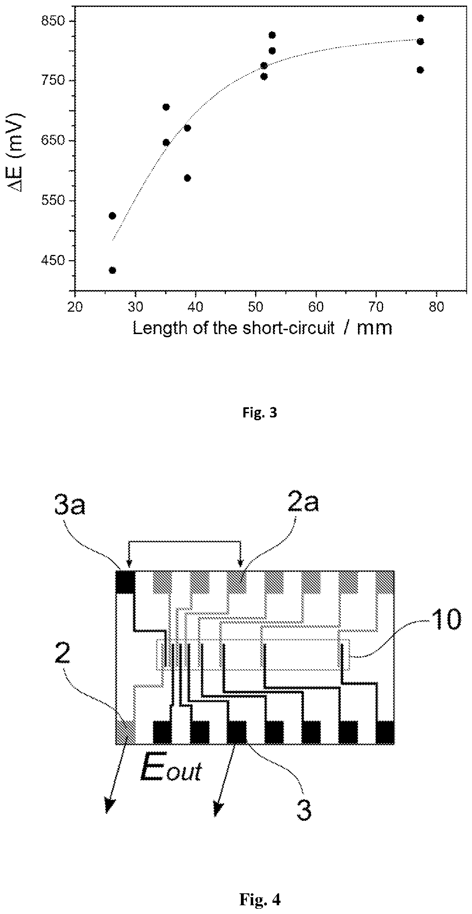

The dependency of the change in the output voltage on the length of the galvanic short-circuit (distance of the inner electrodes cathode 2a - anode 3a) in the environment of 1 mM of $CuSO_4$ is shown in Fig. 3. The quantity $\Delta E$ represents the difference of $E_{out}$ with the short-circuit and $E_{out}$ without the short-circuit, i.e. $E_{cell}$ ($\Delta E = E_{out, with short-circuit} - E_{cell}$). The output voltage increases with increasing distance, after exceeding the distance of $\approx 50$ mm, the output voltage remains substantially unchanged and stabilizes at min. 95% of two times the thermodynamic voltage of the cell. The saturation distance of the connection according to Fig. 1 calculated by interpolation of the fit function of the measured values (shown in Figure 3) is 53 mm.

The output voltage values for different lengths of the short-circuits shown in Fig. 3 (i.e. the values of the output voltage for the connection according to Fig. 1) were measured by means of the electrolytic short-circuit test device, which is schematically shown in Fig. 4. According to this scheme, a pattern etched on a printed circuit board having a lower row of electrodes and an upper row of electrodes was prepared. The upper row of electrodes comprises copper electrodes and one zinc electrode and the bottom row of electrodes comprises zinc electrodes and one copper electrode. An electrolyte of 1 mM $CuSO_4$ in water was applied to the central part of the pattern 10. The output voltage $E_{out}$ of the multiple cell was measured so that the main pair of electrodes, i.e. the copper 2 and one of zinc 3 electrodes in the lower row served for measuring the output voltage and the second pair of inner electrodes, i.e. the zinc 3a and the respective copper 2a electrode in the upper row formed the galvanic short-circuit (they were interconnected by the conductor) so as to maintain the distance of the main electrode from the corresponding electrode of the galvanic short-circuit (e.g. $E_{out}$ for the galvanic short-circuit at the distance of the third position on the test plate in Fig. 4).

### Example 2

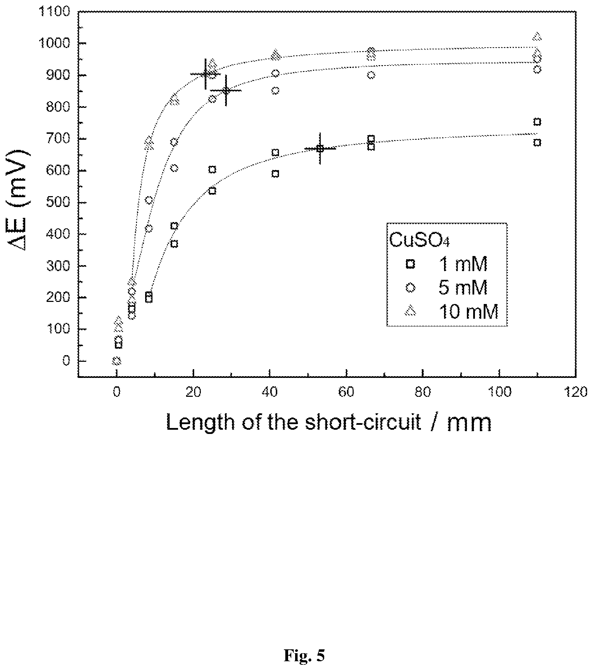

The saturation distance for the galvanic cell system is demonstrated for Daniell cell - a combination of copper and zinc electrodes in the environment of various $CuSO_4$ electrolyte concentrations. Fig. 5 shows the dependency of the change of the output voltage on the length of the galvanic short-circuit, respectively on the distance of the electrodes of the galvanic short-circuit for various concentrations of $CuSO_4$ electrolyte, wherein the calculated values of the saturation distance for concentrations of 1 mM, 5 mM and 10 mM of $CuSO_4$ being 53 mm, 29 mm and 23 mm; the values being displayed by a cross for the given experiment.

Example 3

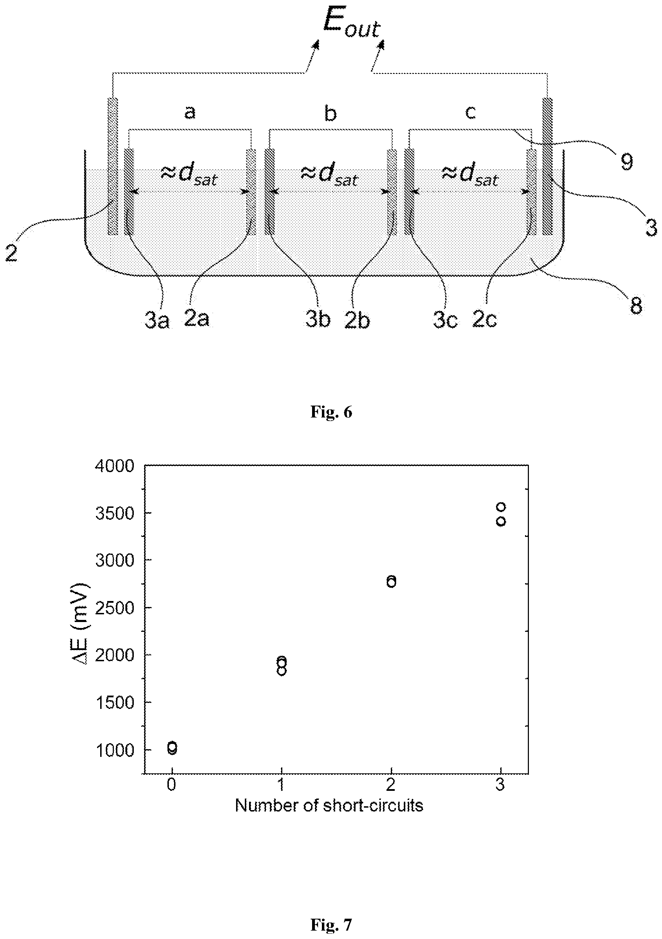

The output voltage of the galvanic system increases linearly with the increasing number of galvanic short-circuits whereas the short-circuit distance remains constant (Fig. 7). The schematic representation of connection of the galvanic system with one (a), two (a + b) and three (a + b + c) galvanic short-circuits connected in the common volume of the electrolyte is shown in Fig. 6. In this embodiment, a constant distance of galvanic short-circuits of 20 mm, a $CuSO_4$ concentration of 10 mM, and the material of the anode and the cathode material as in Example 1 were used.

In Fig. 7, values of the output voltage of a galvanic system comprising one basic cell, i.e. without a short-circuit, one short-circuit (short-circuit connection "a"), two short-circuits (short-circuits connection "a + b") or three short-circuits (short-circuits connection "a + b + c"), wherein each short-circuit value was measured three times. As can be seen in Fig. 7, the output voltage linearly increases with the increasing number of galvanic short-circuits and reaches values that in this particular case exceed 90% of the respective $n$-times the thermodynamic voltage. The connection does not exhibit optimal behavior (95% of the $n$-times the thermodynamic voltage at the saturation distance of the short-circuit), becasue the length of the short-circuit that was used was shorter than the saturation distance for this particular connection - the length of the short-circuit was 20 mm and the saturation distance for the given conditions was 23 mm.

Example 4

In this example, a basic embodiment of the invention is described with the connection of two galvanic cells in a common volume of an electrolyte, as in Fig. 1, wherein the connected galvanic cells are based on the Leclancheé cell, i.e. $MnO_2$ cathode and zinc anode. The $NH_4C1/KOH$ solution is used as electrolyte in this connection, and it is common to both connected cells, i.e. for all electrodes. The $MnO_2$ cathode is composed of a mixture of 5 mg of powdered $MnO_2$, 5 mg of powdered graphite, and 20 $\mu$l of dissolved polymethylmethacrylate in 99.9% acetic acid.

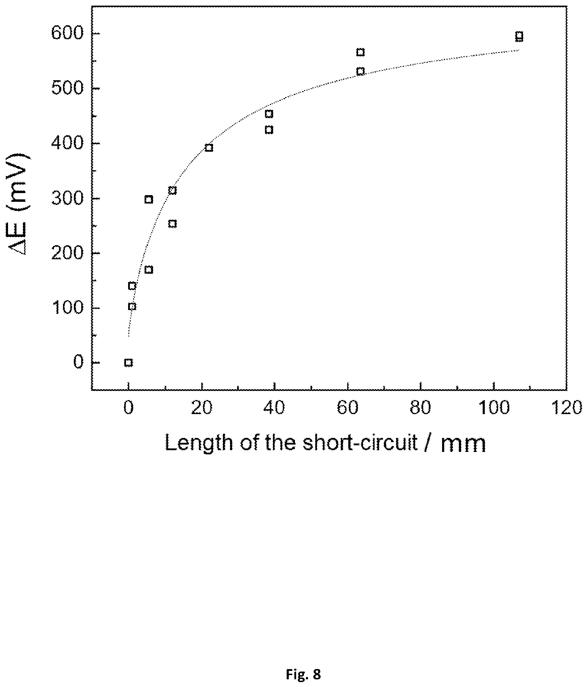

Fig. 8 illustrates the effect of the galvanic short-circuit in a given galvanic system. The output voltage was measured for various short-circuit lengths in the range of 0 mm - 120 mm. Subsequently, the saturation distance of this connection was calculated from the fit function of the measured values. For the electrolyte of 100 mM $NH_4C1/2M$ KOH, the calculated saturation distance of the galvanic short-circuit (i.e., the distance of the inner pair of electrodes) in this connection is ≥192 mm, i.e. at this distance, the electrolytic short-circuit almost does not apply.

Example 5

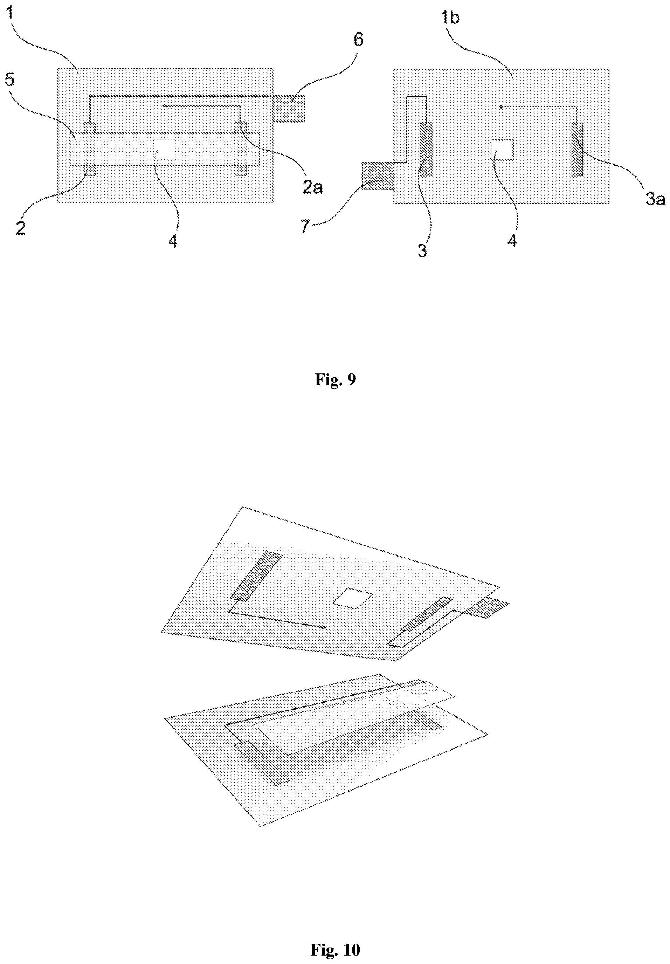

One of the possible practical solutions is a simplified Daniell cell, where copper cathodes and zinc anodes are in the crystalline $CuSO_4$ environment. Such a solution is, for example, a carrier, pre-impregnated with an electrolyte, particularly in this example a filter paper, pre-impregnated with the $CuSO_4$ solution, dried and in contact with the electrodes (Figs. 9 and 10). The energy is only supplied when the filter paper is impregnated with water or other liquid medium, in which crystalline $CuSO_4$ dissolves. Such a galvanic cell can be retained full (charged) as a potential source of energy for almost unlimited period of time, provided the contact with moisture is prevented. This is an advantage compared to conventional technologies, where a spontaneous discharge occurs.

A schematic view of the power supply device described above is shown in Fig. 9 and 10. The device comprises a printed circuit board 1 with a copper contacts and a $CuSO_4$-impregnated filter paper 5. The device further comprises a printed circuit board 1b with electrogalvanized electrodes with a window 4 for the application of water or another liquid medium. The copper electrodes of the first printed circuit board 1 in this embodiment represent cathodes 2, 2a and the zinc galvanized electrodes of the second board 1b of the printed circuit represent anodes 3, 3a. The plates further comprise contacts 6 and 7 for the output voltage take-off. The procedure for assembly of the above described power supply device is shown in Fig. 10 - the printed circuit boards 1 and 1b are assembled so that the cathodes 2, 2a and the anodes 3, 3a touch the filter paper 5 which is impregnated with $CuSO_4$ and forms the common electrolyte for the cathodes 2, 2a and the anodes 3, 3a.

Example 6

An electrical energy source with sufficient voltage for implantable biosensors represents a system comprising several electrode systems in a common volume of an electrolyte according to the present invention. The basis of the system in this example is a biofuel cell published in the literature (Energy Environ Sci., 2012,5, 8891-8895). The anode is implemented as a carbon nanotube aggregate on a gold electrode with an immobilized enzyme PQQ-dependent glucose dehydrogenase that catalyzes glucose oxidation. The cathode is realized as an aggregate of carbon nanotubes on a gold electrode modified with oxygen-reducing laccase enzyme. The inner electrodes are interconnected by a conductor of higher conductivity than the conductivity of amorphous carbon, ie $1.25 \times 10^3$ S/m.

This system is capable of delivering voltages of 300-400 mV. By connecting five of such cells according to the present invention, a power source with an output voltage of min. 1.5 V is provided. The pairs of electrodes anode-cathode of the outer and inner cells are integrated as a source of energy for a device - (bio) sensor that is implanted into the patient's body. Since the body fluids of the patient provide the electrolyte in this embodiment, the biofuel cell becomes active only after implantation.

Industrial applicability

The galvanic system of the present invention can be used as a power source for electronic applications that need a voltage greater than 1 to 1.5 V and do not require high currents, or as a single-purpose built-in power source used for a relatively short time (several hours). Such power sources can be used, for example, for simple electronic devices such as point-of-care diagnostic tools, rescue vests as a water-activated energy source, or humidity sensor, such as a flood detector, as a source of energy activable in humid environments.

The galvanic system of the present invention can also be used as fuel or biofuel cells, and subsequently as a source of energy for implantable (bio) sensors (e.g., glucometers) or other electronic devices. The galvanic system of the present invention may also represent a source of energy with the voltage higher than the thermodynamic voltage in the common volume of the electrolyte (in this case the patient's body) for implantable electronic devices without an inserted battery / accumulator when the energy is supplied by the (bio) fuel cell and the substances contained in this electrolyte, directly in the patient's body, represent the fuel.

**Claims**

1. A galvanic system comprising a main pair of electrodes cathode (2) - anode (3) and at least one galvanic short-circuit formed by a pair of inner electrodes cathode (2a) - anode (3a), **characterized in that** the main electrodes (2, 3) and the inner electrodes (2a, 3a) are placed in a common electrolyte (8) and **in that** said at least one galvanic short-circuit is connected between the cathode (2) and the anode (3) of the main pair, wherein each inner cathode (2a) is positioned between the respective inner anode (3a) and the main anode (3), wherein the distance between the inner electrodes (2a, 3a) is greater than or equal to the saturation distance, the saturation distance being defined as the distance between the inner electrodes (2a, 3a), at which the output voltage reaches 95% of the voltage that would be reached if the distance between the inner anode (3a) and the inner cathode (2a) was infinitely large, and wherein the inner cathode (2a) and the inner anode (3a) are interconnected by a conductor (9) of higher conductivity than the conductivity of the electrolyte (8) thereby forming a galvanic short-circuit.

2. The galvanic system according to claim 1, **characterized in that** it comprises at least two galvanic short-circuits, said at least two galvanic short-circuits being connected between the cathode (2) and the anode (3) of the main pair, wherein the inner cathode (2a, 2b) of each connected galvanic short-circuit is positioned between the inner anode (3a, 3b) of the respective galvanic short-circuit and the main anode (3), and wherein the individual inner galvanic short-circuits do not overlap.

3. The galvanic system according to any one of the preceding claims, **characterized in that** it further comprises an electrolyte carrier (5), **in that** the main electrodes (2, 3) and inner electrodes (2a, 3a) are in contact with the electrolyte carrier (5) and **in that** the electrolyte (8) is in a solid form without the presence of a solvent.

4. The galvanic system according to any one of the preceding claims, **characterized in that** it comprises copper cathodes (2, 2a), zinc anodes (3, 3a) and electrolyte containing $CuSO_4$, or cathodes (2, 2a) containing $MnO_2$, zinc anodes (3, 3a) and electrolyte containing $NH_4Cl/KOH$.

5. A method of increasing the output voltage of a galvanic system by combining several cells in a common volume of electrolyte, **characterized in that** it comprises connecting electrodes of main pair cathode (2) - anode (3) into an electrolyte (8), connecting at least one galvanic short-circuit formed by a pair of inner electrodes cathode (2a) - anode (3a) into the same electrolyte (8) so that the inner cathode (2a) is positioned between the respective inner anode (3a) and the main anode (3), wherein the distance between the inner electrodes (2a, 3a) is greater than or equal to the saturation distance, the saturation distance being defined as the distance between the inner electrodes (2a, 3a), at which the output voltage reaches 95% of the voltage that would be reached if the distance between the inner anode (3a) and the inner cathode (2a) was infinitely large, and wherein the individual inner galvanic short-circuits do not overlap, and interconnecting the inner electrodes (2a, 3a) with a conductor (9) of higher conductivity than the conductivity of the electrolyte (8).

6. The method of increasing the output voltage according to claim 5, **characterized in that** an electrolyte carrier (5) is provided before the electrodes of the main pair cathode (2) - anode (3) are connected into the electrolyte (8), the carrier (5) is impregnated with the electrolyte (8), and the carrier (5) impregnated with the electrolyte (8) is dried such that the electrolyte (8) remains on the carrier in a solid form without the presence of a solvent.

7. A fuel cell comprising a galvanic system according to any one of claims 1 to 3.

8. A biofuel cell comprising a galvanic system according to any one of claims 1 to 3.

9. A device for measuring body functions or parameters, **characterized in that** it is adapted for implantation into a human body, **in that** it comprises a galvanic system with a main pair of electrodes cathode (2) - anode (3) and at least one galvanic short-circuit formed by a pair of inner electrodes cathode (2a) - anode (3a), wherein the main electrodes (2, 3) and the inner electrodes (2a, 3a) are arranged for placement in a common electrolyte, and **in that** said at least one galvanic short-circuit is connected between the cathode (2) and the anode (3) of the main pair, wherein each inner cathode (2a) is positioned between the respective inner anode (3a) and the main anode (3), wherein the distance between the inner electrodes (2a, 3a) is greater than or equal to the saturation distance, the saturation distance being defined as the distance between the inner electrodes (2a, 3a), at which the output voltage reaches 95% of the voltage that would be reached if the distance between the inner anode (3a) and the inner cathode (2a) was infinitely large, wherein the individual inner galvanic short-circuit(s) do not overlap, and wherein the inner cathode (2a) and the inner anode (3a) are interconnected by a conductor (9) with a higher conductivity than the conductivity of amorphous carbon, thereby forming the galvanic short-circuit.

**Patentansprüche**

1. Galvanisches System, umfassend ein Hauptpaar von Elektroden Kathode (2) - Anode (3) und mindestens einen galvanischen Kurzschluss, der durch ein Paar von Innenelektroden Kathode (2a) - Anode (3a) gebildet wird, **dadurch gekennzeichnet, dass** die Hauptelektroden (2, 3) und die inneren Elektroden (2a, 3a) sind in einem gemeinsamen Elektrolyten (8) angeordnet, und wobei der mindestens eine galvanische Kurzschluss zwischen der Kathode (2) und der Anode (3) von das Hauptpaar geschaltet ist, wobei jede innere Kathode (2a) zwischen der jeweiligen inneren Anode (3a) und der Hauptanode (3) positioniert ist, wobei der Abstand zwischen den inneren Elektroden (2a, 3a) größer oder gleich dem Sättigungsabstand ist, wobei der Sättigungsabstand als der Abstand zwischen den inneren Elektroden (2a, 3a) definiert ist, bei dem die Ausgangsspannung 95% der Spannung erreicht, die erreicht werden würde, wenn der Abstand zwischen der inneren Anode (3a) und der inneren Kathode (2a) unendlich groß wäre, und wobei die innere Kathode (2a) und die innere Anode (3a) miteinander verbunden sind durch einen Leiter (9) mit höherer Leitfähigkeit als die Leitfähigkeit des Elektrolyten (8), wodurch ein galvanischer Kurzschluss entsteht.

2. Galvanisches System nach Anspruch 1, **dadurch gekennzeichnet, dass** es mindestens zwei galvanische Kurzschlüsse umfasst, wobei die mindestens zwei galvanische Kurzschlüsse zwischen der Kathode (2) und der Anode (3) des Hauptpaars angeschlossen sind; wobei die innere Kathode (2a, 2b) jedes angeschlossenen galvanischen Kurzschlusses zwischen der inneren Anode (3a, 3b) des jeweiligen galvanischen Kurzschlusses und der Hauptanode (3) positioniert ist und wobei die einzelne innere galvanische Kurzschlüsse überlappen sich nicht.

3. Galvanisches System nach einem der vorhergehenden Ansprüche, **dadurch gekennzeichnet, dass** es ferner einen Elektrolytträger (5) umfasst, indem die Hauptelektroden (2, 3) und Innenelektroden (2a, 3a) mit dem Elektrolytträger (5) in Kontakt stehen, und wobei der Elektrolyt (8) in fester Form ohne die Anwesenheit eines Lösungsmittels ist.

4. Galvanisches System nach einem der vorhergehenden Ansprüche, **dadurch gekennzeichnet, dass** es Kupferkathoden (2, 2a), Zinkanoden (3, 3a) und $CuSO_4$ enthaltenden Elektrolyt umfasst, oder $MnO_2$ enthaltende Kathoden (2, 2a), Zink Anoden (3, 3a) und $NH_4C1/KOH$ enthaltenden Elektrolyt umfasst.

5. Ein Verfahren zum Erhöhen der Ausgangsspannung eines galvanischen Systems durch Kombinieren mehrerer Zellen in einem gemeinsamen Elektrolytvolumen, das **dadurch gekennzeichnet ist, dass** es Verbinden von Elektroden der Hauptpaarkathode (2) - Anode (3) zu einem Elektrolyten (8) umfasst; Verbinden mindestens eines galvanischen Kurzschlusses, der durch ein Paar von Innenelektroden Kathode (2a) - Anode (3a) gebildet wird, mit demselben Elektrolyten (8), so dass die innere Kathode (2a) zwischen der jeweiligen inneren Anode (3a) und die Hauptanode (3) positioniert ist, wobei der Abstand zwischen den inneren Elektroden (2a, 3a) größer oder gleich dem Sättigungsabstand ist, wobei der Sättigungsabstand als der Abstand zwischen den inneren Elektroden (2a, 3a) definiert ist, bei dem die Ausgangsspannung erreicht 95% der Spannung, die erreicht werden würde, wenn der Abstand zwischen der inneren Anode (3a) und der inneren Kathode (2a) unendlich groß wäre, und wobei sich die einzelne innere galvanische Kurzschlüsse nicht überlappen, und Verbinden von den inneren Elektroden (2a, 3a) mit einen Leiter (9) mit höherer Leitfähigkeit als die Leitfähigkeit des Elektrolyten (8).

6. Verfahren zum Erhöhen der Ausgangsspannung nach Anspruch 5, **dadurch gekennzeichnet, dass** ein Elektrolytträger (5) vorgesehen ist, bevor die Elektroden der Hauptpaar Kathode (2) - Anode (3) mit dem Elektrolyten (8) verbunden sind; der Träger (5) wird mit dem Elektrolyten (8) imprägniert, und der mit dem Elektrolyten (8) imprägnierte Träger (5) wird so getrocknet, dass der Elektrolyt (8) in fester Form ohne Anwesenheit eines Lösungsmittels auf dem Träger verbleibt.

7. Brennstoffzelle, umfassend ein galvanisches System nach einem der Ansprüche 1 bis 3.

8. Biobrennstoffzelle, umfassend ein galvanisches System nach einem der Ansprüche 1 bis 3.

9. Vorrichtung zum Messen von Körperfunktionen oder -parametern, die **dadurch gekennzeichnet ist, dass** sie zur Implantation in einen menschlichen Körper geeignet ist, wobei sie ein galvanisches System mit einem Hauptpaar von Elektroden, Kathode (2) - Anode (3), und mindestens einer galvanischer Kurzschluss, gebildet durch ein Paar von Innenelektroden Kathode (2a) - Anode (3a), umfasst, wobei die Hauptelektroden (2, 3) und die Innenelektroden (2a, 3a) zur Platzierung in einem gemeinsamen Elektrolyten angeordnet sind, und wobei der mindestens ein galvanischer Kurzschluss zwischen der Kathode (2) und der Anode (3) des Hauptpaars verbunden ist, wobei jede innere Kathode (2a) zwischen der jeweiligen inneren Anode (3a) und der Hauptanode (3) positioniert ist, wobei der Abstand zwischen den inneren Elektroden (2a, 3a) größer oder gleich dem Sättigungsabstand ist, wobei der Sättigungsabstand als der Abstand zwischen den inneren Elektroden (2a, 3a) definiert ist, bei dem die Ausgangsspannung erreicht 95% der Spannung, die erreicht werden würde, wenn der Abstand zwischen der inneren Anode (3a) und der inneren Kathode (2a) unendlich groß wäre, wobei sich die einzelnen inneren galvanischen Kurzschlüsse nicht überlappen und wobei die innere Kathode (2a) und die innere Anode (3a) durch einen Leiter miteinander verbunden sind (9) mit einer höheren Leitfähigkeit als die Leitfähigkeit von amorphem Kohlenstoff, wodurch der galvanische Kurzschluss gebildet wird.

## Revendications

1. Système galvanique comprenant une paire d'électrodes principales cathode (2) - anode (3) et au moins un court-circuit galvanique formé par une paire d'électrodes internes cathode (2a) - anode (3a), **caractérisé en ce que** les électrodes principales (2, 3) et les électrodes internes (2a, 3a) sont placées dans un électrolyte commun (8) et **en ce que** ledit au moins un court-circuit galvanique est connecté entre la cathode (2) et l'anode (3) de la paire principale, où chaque cathode interne (2a) est positionnée entre l'anode interne respective (3a) et l'anode principale (3), où la distance entre les électrodes internes (2a, 3a) est supérieure ou égale à la distance de saturation, la distance de saturation étant définie comme la distance entre les électrodes internes (2a, 3a), à laquelle la tension de sortie atteint 95% de la tension qui serait atteinte si la distance entre l'anode interne (3a) et la cathode interne (2a) était infiniment grande, et où la cathode interne (2a) et l'anode interne (3a) sont interconnectées par un conducteur (9) de conductivité supérieure à la conductivité de l'électrolyte (8) formant ainsi un court-circuit galvanique.

2. Système galvanique selon la revendication 1, **caractérisé en ce qu'**il comprend au moins deux courts-circuits galvaniques, lesdits au moins deux courts-circuits galvaniques étant connectés entre la cathode (2) et l'anode (3) de la paire principale, où la cathode interne (2a, 2b) de chaque court-circuit galvanique connecté est positionnée entre l'anode interne (3a, 3b) du court-circuit galvanique respectif et l'anode principale (3), et où le court-circuits galvaniques internes individuels ne se chevauchent pas.

3. Système galvanique selon l'une quelconque des revendications précédentes, **caractérisé en ce qu'**il comprend en outre un support d'électrolyte (5), **en ce que** les électrodes principales (2, 3) et les électrodes internes (2a, 3a) sont en contact avec le support d'électrolyte (5) et **en ce que** l'électrolyte (8) est sous forme solide sans la présence d'un solvant.

4. Système galvanique selon l'une quelconque des revendications précédentes, **caractérisé en ce qu'**il comprend des cathodes (2, 2a) en cuivre, des anodes (3, 3a) en zinc et un électrolyte contenant du $CuSO_4$, ou des cathodes (2, 2a) contenant du $MnO_2$, des anodes (3, 3a) en zinc et un électrolyte contenant $NH_4Cl/KOH$.

5. Procédé d'augmentation de la tension de sortie d'un système galvanique en combinant plusieurs cellules dans un même volume d'électrolyte, **caractérisé en ce qu'**il comprend la connexion d'électrodes de la paire principale cathode (2) - anode (3) dans un électrolyte (8), connexion d'au moins un court-circuit galvanique formé par une paire d'électrodes internes cathode (2a) - anode (3a) dans le même électrolyte (8) de sorte que la cathode interne (2a) soit positionnée entre l'anode interne respective (3a) et l'anode principale (3), où la distance entre les électrodes internes (2a, 3a) est supérieure ou égale à la distance de saturation, la distance de saturation étant définie comme la distance entre les électrodes internes (2a, 3a), à laquelle la tension de sortie atteint 95% de la tension qui serait atteinte si la distance entre l'anode interne (3a) et la cathode interne (2a) était infiniment grande, et où les courts-circuits galvaniques internes individuels ne se chevauchent pas, et interconnexion d'électrodes internes (2a, 3a) avec un conducteur (9) de conductivité supérieure à la conductivité de l'électrolyte (8).

6. Procédé d'augmentation de la tension de sortie selon la revendication 5, **caractérisé en ce qu'**un support d'électrolyte (5) est prévu avant que les électrodes de la paire principale cathode (2) - anode (3) ne soient connectées à l'électrolyte (8), le support (5) est imprégné de l'électrolyte (8), et le support (5) imprégné de l'électrolyte (8) est séché de telle sorte que l'électrolyte (8) reste sur le support sous forme solide sans la présence d'un solvant.

7. Pile à combustible comprenant un système galvanique selon l'une quelconque des revendications 1 à 3.

8. Pile à biocombustible comprenant un système galvanique selon l'une quelconque des revendications 1 à 3.

9. Dispositif de mesure de fonctions ou de paramètres corporels, **caractérisé en ce qu'**il est adapté pour une implantation dans un corps humain, **en ce qu'**il comprend un système galvanique avec une paire principale d'électrodes cathode (2) - anode (3) et au moins une court-circuit galvanique formé par une paire d'électrodes internes cathode (2a) - anode (3a), où les électrodes principales (2, 3) et les électrodes internes (2a, 3a) sont disposées pour être placées dans un électrolyte commun, et dans **en ce que** ledit au moins un court-circuit galvanique est connecté entre la cathode (2) et l'anode (3) de la paire principale, dans lequel chaque cathode interne (2a) est positionnée entre l'anode interne respective (3a) et l'anode principale (3), où la distance entre les électrodes internes (2a, 3a) est supérieure ou égale à la distance de saturation, la distance de saturation étant définie comme la distance entre les électrodes internes (2a, 3a), à laquelle la tension de sortie atteint 95 % de la tension de sortie qui serait atteinte si la distance entre l'anode interne (3a) et la cathode interne (2a) était infiniment grande, où le(s) court-circuits) galvanique(s) interne(s) ne se chevauchent pas, et dans lequel la cathode interne (2a) et l'anode interne (3a) sont interconnectées par un conducteur (9) avec une conductivité plus élevée que la conductivité du carbone amorphe, formant ainsi le court-circuit galvanique.

**Fig. 1**

Fig. 2

**Fig. 3**

**Fig. 4**

**Fig. 5**

**Fig. 6**

**Fig. 7**

Fig. 8

**Fig. 9**

**Fig. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Energy Environ Sci.*, 2012, vol. 5, 8891-8895